(19) Europäisches Patentamt
European Patent Office
Office européen des brevets

(11) **EP 3 134 540 B1**

(12) **EUROPEAN PATENT SPECIFICATION**

(45) Date of publication and mention
of the grant of the patent:
**25.07.2018 Bulletin 2018/30**

(21) Application number: **14719023.5**

(22) Date of filing: **24.04.2014**

(51) Int Cl.:
***C12Q 1/68*** (2018.01)

(86) International application number:
**PCT/EP2014/058356**

(87) International publication number:
**WO 2015/161880 (29.10.2015 Gazette 2015/43)**

(54) **AN IN VITRO DIAGNOSTIC METHOD FOR CANCER BY MEANS OF CIRCULATING, CELL-FREE DNA**

IN-VITRO-DIAGNOSEVERFAHREN FÜR KREBS MITTELS ZIRKULIERENDER ZELLFREIER DNA

MÉTHODE IN VITRO DE DIAGNOSTIC DU CANCER UTILISANT L'ADN CIRCULANT EXEMPT DE CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**01.03.2017 Bulletin 2017/09**

(73) Proprietor: **Euroclone Spa**
**20016 Pero (Milano) (IT)**

(72) Inventors:
  • **SACCANI, Andrea**
    **I-20149 Milano (IT)**
  • **AGOSTINI, Marco**
    **I-35035 Mestrino (IT)**
  • **BEDIN, Chiara**
    **I-36043 Camisano Vicentino (IT)**

(74) Representative: **Croce, Valeria**
    **Jacobacci & Partners S.p.A.**
    **Via Senato, 8**
    **20121 Milano (IT)**

(56) References cited:
  • MARIANGELA ZANE ET AL: "Circulating cell-free DNA, SLC5A8 and SLC26A4 hypermethylation, BRAFV600E: A non-invasive tool panel for early detection of thyroid cancer", BIOMEDICINE & PHARMACOTHERAPY, vol. 67, no. 8, 1 October 2013 (2013-10-01), pages 723-730, XP055163365, ISSN: 0753-3322, DOI: 10.1016/j.biopha.2013.06.007

  • FENG JIANG ET AL: "Plasma cell-free DNA and its DNA integrity as biomarker to distinguish prostate cancer from benign prostatic hyperplasia in patients with increased serum prostate-specific antigen", INTERNATIONAL UROLOGY AND NEPHROLOGY, AKADEMIAI, BUDAPEST, HU, vol. 45, no. 4, 19 June 2013 (2013-06-19), pages 1023-1028, XP035374285, ISSN: 0301-1623, DOI: 10.1007/S11255-013-0491-2 [retrieved on 2013-06-19]

  • AGOSTINI M ET AL: "Circulating cell-free DNA: a promising marker of regional lymphonode metastasis in breast cancer patients.", CANCER BIOMARKERS : SECTION A OF DISEASE MARKERS 2012, vol. 11, no. 2-3, 2012, pages 89-98, XP008174324, ISSN: 1875-8592

  • PÂMELA OLIVEIRA DELGADO ET AL: "Characterization of cell-free circulating DNA in plasma in patients with prostate cancer", TUMOR BIOLOGY, vol. 34, no. 2, 27 December 2012 (2012-12-27), pages 983-986, XP008174318, ISSN: 1010-4283, DOI: 10.1007/s13277-012-0634-6

  • MARZESE D M ET AL: "Diagnostic and prognostic value of circulating tumor-related DNA in cancer patients", EXPERT REVIEW OF MOLECULAR DIAGNOSTICS 2013 EXPERT REVIEWS LTD. GBR, vol. 13, no. 8, 2013, pages 827-844, XP008174327, ISSN: 1473-7159

EP 3 134 540 B1

- **TANAKA H ET AL: "Role of circulating free Alu DNA in endometrial cancer", INTERNATIONAL JOURNAL OF GYNECOLOGICAL CANCER 2012 LIPPINCOTT WILLIAMS AND WILKINS USA, vol. 22, no. 1, January 2012 (2012-01), pages 82-86, XP008174316, ISSN: 1048-891X**
- **H. SCHWARZENBACH ET AL: "Cell-free Tumor DNA in Blood Plasma As a Marker for Circulating Tumor Cells in Prostate Cancer", CLINICAL CANCER RESEARCH, vol. 15, no. 3, 1 February 2009 (2009-02-01), pages 1032-1038, XP055083221, ISSN: 1078-0432, DOI: 10.1158/1078-0432.CCR-08-1910**
- **MARCO AGOSTINI ET AL: "Circulating Cell-Free DNA: A Promising Marker of Pathologic Tumor Response in Rectal Cancer Patients Receiving Preoperative Chemoradiotherapy", ANNALS OF SURGICAL ONCOLOGY, SPRINGER-VERLAG, NE, vol. 18, no. 9, 17 March 2011 (2011-03-17), pages 2461-2468, XP019945160, ISSN: 1534-4681, DOI: 10.1245/S10434-011-1638-Y**
- **ALIX-PANABIERES CATHERINE ET AL: "Circulating Tumor Cells and Circulating Tumor DNA", ANNUAL REVIEW OF MEDICINE, VOL 63 ANNUAL REVIEWS, 4139 EL CAMINO WAY, PO BOX 10139, PALO ALTO, CA 94303-0897 USA SERIES : ANNUAL REVIEW OF MEDICINE (ISSN 0066-4219(PRINT)), 2012, pages 199-215, XP002734777,**
- **YAHYA ELSHIMALI ET AL: "The Clinical Utilization of Circulating Cell Free DNA (CCFDNA) in Blood of Cancer Patients", INTERNATIONAL JOURNAL OF MOLECULAR SCIENCES, vol. 14, no. 9, 13 September 2013 (2013-09-13), pages 18925-18958, XP055163790, DOI: 10.3390/ijms140918925**

**Description**

The present invention relates to an *in vitro* method for the diagnosis of prostate cancer.

It is known that free DNA and DNA sequences can be found in body fluids as a result of the apoptosis process. Differently from the sequences released by normal cells, there can be also found altered sequences which are indicative of the presence of altered, i.e. tumoral, cells.

The identification and possibly the quantification of such sequences, therefore, represents a way to identify the presence of a cancer in a subject and therefore a potentially powerful diagnostic tool.

The publication of Mariangela Zane et al ("Circulating cell-free DNA, SLC5A8 and SLC26A4 hypermethylation, BRAFV600E: A non-invasive tool panel for early detection of thyroid cancer", Biomedicine & Pharmacotherapy, vol. 67, no.8, 1 October 2013, pages 723-730) discloses the correlation of cell free DNA corresponding to the sequences ALU 244 and ALU 83 with the cancer to thyroid.

The publication of Feng Jiang et al ("Plasma cell-free DNA and its DNA integrity as biomarker to distinguish prostate cancer from benign prostatic hyperplasia in patients with increased serum prostate-specific antigen", International urology and nephrology, Akademiai, Budapest, HU, vol. 45, no. 4, 19 June 2013, pages 1023-1028) discloses that the levels of free DNA in the plasma may be used to distinguish prostate cancer from benign tumors and, in particular, it cites the sequences ALE 247 and ALU 115 and their relative ratio as markers.

Summary of the invention

The present invention discloses a method for the *in vitro* diagnosis of prostate cancer, wherein specific ALU sequences are detected in a body sample.

Object of the invention

A first object of the invention is represented by an *in vitro* method for the diagnosis of prostate cancer. In a further aspect, the invention discloses a method for the prognosis of prostate cancer.

Detailed description of the invention

*Definitions*

For the purposes of the present invention, a sample is an isolated aliquot of a body fluid of a subject.

In particular, the sample used is a sample of plasma, serum, saliva, urine, fluid from lymph nodes or lymph organs, bone marrow, fluid from pleura, breast ducts, lacrimal fluid, serous fluid, peritoneal fluid, liquor (cerebral spinal fluid), stool, sputum, ascites, gastric or pancreatic juice, sweat.

In a preferred embodiment of the invention the sample is an isolated sample of blood or serum or liquor sample, whereas in a most preferred sample is an isolated sample of blood or serum.

The volume of the sample suitable for performing the method of the invention depends upon the type of the body fluid.

In particular, a suitable volume is comprised between about 300 $\mu$l-1 ml and preferably is about 500 $\mu$l.

In a first aspect of the invention, the blood sample is not purified.

Accordingly, once taken from the subject, the only steps performed comprise the centrifugation and/or filtration in order to remove the particulate (cells). In addition, other suitable treatments can be performed on the sample in order to remove or inactivate proteins, like for instance a step of treatment with enzymes.

In another embodiment of the invention, the sample is subjected to a step for the DNA extraction.

Said step can be performed according to well-known techniques in the art, like for instance the extraction with phenol/chloroform.

For the purposes of the present invention, a subject is intended to be a human or an animal.

Within animals, pets and courtyard animals are preferred, wherein mammals are particularly preferred. In an even preferred embodiments, animals refers to dogs.

The "total circulating DNA" according to the invention can be determined with quantitative PCR or other method suitable in the art.

Within the present invention, the "amount of" is used to refer to a value which is representative of the concentration of a particular sequence (nucleic sequence) in the sample.

"Representative" is used to refer to a relation between a two values.

For instance, a measurement of a compound in a sample could provide a value which can be used to determine the concentration through a known calculation.

The way to calculate the concentration from the first value depends upon the circumstances as well as upon the method used.

The "DNA integrity" refers to the ratio of the amount of the circulating DNA released from cancer cells to the total amount of DNA.

In the diagnostic method of the invention, the subject is a person or an animal likely to develop cancer or another cancer (i.e. recurrence of the cancer) or suspected of having cancer.

The *in vitro* diagnostic method of the present invention comprises in particular the steps of:

1) determining the total circulating DNA in a sample;
2) determining the DNA released by cancer cells;
3) determining the value "II" (Integrity Index), which corresponds to the ratio between the values of steps 1) and 2).

In particular, the step 1) comprises determining the amount of ALU 83 present in the sample.

For said purpose, the following primers are used:

| | Sequence 5'→3' : | SEQ. ID. n. |
|---|---|---|
| forward | CTGAGGTCAGGAGTTCGAGACC | 1 |
| reverse | CCACGCCCGGCTAATTTT | 2 |

As for step 2), it comprises determining the amount of ALU 244 present in the sample.

For said purpose, the following primers are used:

| | Sequence 5'→3' : | SEQ. ID. n. |
|---|---|---|
| forward | GCGGTGGCTCACGCCTGTAA | 3 |
| reverse | GGAGTGCAGTGGCGCGATCT | 4 |

In step 3) the ratio "II" between the amount of the DNA released by cancer cells and the total circulating DNA is calculated, as it can be represented below:

$$II = \frac{ALU244\ value}{ALU83\ value}$$

In a preferred embodiment "value" stands for a measure of quantity, like concentration, determined with quantitative PCR; therefore, the "C" value is calculated as below:

$$C = \frac{ALU244-qPCR\ value}{ALU83-qPCR\ value}$$

According to a preferred embodiment of the present invention, in the above steps 1) and 2) the ALU sequences are amplified by quantitative PCR (qPCR).

In addition to the above steps 1) to 3), the in vitro diagnostic method of the invention comprises the additional step of comparing the data obtained for the "II" value with a pool of reference values.

For the purposes of the present invention a pool of reference values comprises values which are known to correspond to either:

- health patients, or
- patients with cancer, or
- to a disease state of a group of patients.

In particular, the reference value depends upon characteristics of the patient undergoing the *in vitro* diagnostic method of the invention, like age, sex, race, etc.

In addition to that, the reference value depends upon the kind of sample under examination and upon the type of tumor which is investigated.

In the preferred embodiment, the method above disclosed allows the diagnosis of the prostate cancer. According

4

to another object of the invention, there is disclosed a method of treatment comprising the step of performing the *in vitro* diagnostic method of the invention.

In particular, said method can be used for the treatment of cancer and, more in particular, of the prostate cancer.

More in detail, said method can be performed on a subject who developed a cancer or who is being treated against a cancer or who underwent treatment against cancer, like a surgical intervention to remove cancer. Accordingly, the present method can be used for monitoring the progression of the cancer as well as the response to the therapeutic treatment.

In fact, any variation in the data collected can be indicative of the progression or regression of the cancer in case the therapeutic treatment is ineffective or successful.

Therefore, the method of the invention can also be used for identifying if a drug or a treatment is effective against cancer.

In another object of the invention, there is disclosed a method for the prognosis of the cancer comprising carrying out the method of the invention.

For said purpose, the method can be performed on a subject which is likely to develop a cancer in the future.

In this regard, consideration shall be done on the environment the subject lives in (whether in the past there has been contact with potentially dangerous substances) and/or on their familiarity with the pathology (for instance, if one or more close relatives developed a cancer).

The data of the "C" value can be collected at one or subsequent times and compared to reference values, which are indicative of the onset of the pathology. Also in this case, the reference data depends upon characteristics of the patient undergoing the method of the invention, like age, sex, race, etc.

In addition to that, the reference data depends upon the kind of sample under examination and upon the type of tumor which is investigated.

Thanks to the method of the invention, the curative treatment of the patient can be advantageously started at a very early stage of the pathology.

The methods above disclosed which make use of the *in vitro* diagnostic method of the invention can be used in combination with other method so as to achieve even more reliable and effective results.

In accordance with a further object of the present invention, it is provided a kit for performing the *in vitro* diagnostic method of the invention.

In particular, said kit comprises:

- primers for the amplification of ALU 83; and

- primers for the amplification of ALU 244.

More in detail, each of the primers is included in an appropriate reaction solution comprising a suitable probe.

In a first embodiment of the invention, a reactant for amplification of ALU83 comprises:

| | |
|---|---|
| Fluocycle II ™ Master Mix probe (without ROX) (Euroclone S.p.A.) | 10 $\mu$l |
| ALU83 fwd (10 $\mu$M) | 0.1 $\mu$l |
| ALU83 rev (10 $\mu$M) | 1.8 $\mu$l |
| probe (10 $\mu$M) | 0.5 $\mu$l |
| water | to 20 $\mu$l |

Fluocycle II™ Master Mix formulation comprises: 100 mM KCl, 20 mM Tris HCl pH 8.3, 0.02% Tween-20, 0.8 mM of each dNTPs (dATP, dCTP, dGTP, dTTP), 200 units/ml Taq DNA polymerase, 8 mM MgCl$_2$, stabilizers.

On the other side, the reactant for amplification of ALU244 comprises:

| | |
|---|---|
| Fluocycle II™ Master Mix probe (without ROX) (Euroclone S.p.A.) | 10 $\mu$l |
| ALU244 fwd (10 $\mu$M) | 1.8 $\mu$l |
| ALU244 rev (10 $\mu$M) | 1.8 $\mu$l |
| probe (10 $\mu$M) | 0.5 $\mu$l |
| water | to 20 $\mu$l |

Fluocycle II™ Master Mix formulation comprises: 100 mM KCl, 20 mM Tris HCl pH 8.3, 0.02% Tween-20, 0.8 mM of each dNTPs (dATP, dCTP, dGTP, dTTP), 200 units/ml Taq DNA polymerase, 8 mM MgCl$_2$, stabilizers.

The kit of the invention preferably also comprises a sequence for performing the standard reference curve. For instance, a preparation of an ALU sequence, which can be in a lyophilized form or in solution. According to another aspect, the kit may comprise in addition a sample which can be used to verify the inter-assay variability.

In a first kit of the invention, there are included:

- the above reactant for amplification of ALU83;

- the above reactant for amplification of ALU244;

- the sequence for performing the standard reference curve, such as a preparation of an ALU sequence;

- a sample for verifying the inter-assay variability.

In an alternative kit, there can be included:

- a solution Fluocycle II™ Master Mix probe (without ROX) (Euroclone S.p.A.);

- a solution comprising the primers for the ALU83 sequence and a suitable probe;

- a solution comprising the primers for the ALU244 sequence and a suitable probe;

- the sequence for performing the standard reference curve, such as a preparation of an ALU sequence;

- a reference sample for verifying the inter-assay variability.

In the above kit, the primer solutions for the ALU sequences preferably are:

| | |
|---|---|
| ALU83 fwd (10 $\mu$M) | 0.1 $\mu$l |
| ALU83 rev (10 $\mu$M) | 1.8 $\mu$l |
| probe (10 $\mu$M) water | 0.5 $\mu$l |

and

| | |
|---|---|
| ALU244 fwd (10 $\mu$M) | 1.8 $\mu$l |
| ALU244 rev (10 $\mu$M) | 1.8 $\mu$l |
| probe (10 $\mu$M) water | 0.5 $\mu$l |

The kit can further comprise a collection of data that can be used as a reference in the *in vitro* diagnostic, therapeutic or prognostic method of the invention.

*Materials and methods*

**cfDNA extraction kit**

QIAamp(R) UltraSensTM Virus Kit (QIAGEN):

Starting volume plasma: 500 $\mu$l

cfDNA elution volume: 50 $\mu$l

**Real Time PCR amplification protocol**

**Standard Curve**

| | |
|---|---|
| Standard 6: | 0.5 pg/$\mu$l |
| Standard 5: | 1 pg/$\mu$l |

(continued)

| Standard 4: | 10 pg/$\mu$l |
|---|---|
| Standard 3: | 100 pg/$\mu$l |
| Standard 2: | 1*10$^3$pg/$\mu$l |
| Standard 1: | 10*10$^3$ pg/$\mu$l |

| reactant for mix ALU83 | $\mu$l |
|---|---|
| Fluocycle II Master Mix probe (NO ROX) | 10 |
| ALU83fwd (10 $\mu$M) | 0.1 |
| ALU83rev (10 $\mu$M) | 1.8 |
| probe (10 $\mu$M) | 0.5 |
| H$_2$O | 6.6 |
| **cfDNA/Standard** | **1** |
| volume tot | 20 |

| reactant for mix ALU244 | $\mu$l |
|---|---|
| Fluocycle II Master Mix probe (NO ROX) | 10 |
| ALU244fwd (10 $\mu$M) | 1.8 |
| ALU244rev (10 $\mu$M) | 1.8 |
| probe (10 $\mu$M) | 0.5 |
| H$_2$O | 4.9 |
| **cfDNA/Standard** | **1** |
| volume tot | 20 |

**Amplification protocol for the ALU mix**

| 95°C | 5 min | |
|---|---|---|
| 95°C | 15 sec | X40 cycles |
| 62°C | 1 min | |

**Probe**

6FAM-CCTGGCCAACATGGTGAAACCCC-TMR **SEQ.ID. n.5**

**EXAMPLE 1**

*Quantitative PCR of plasma DNA fragments*

The quantification of the cell free DNA (cf-DNA) fragments in plasma was performed by quantitative real time PCR (qPCR), which amplified and quantified the shortest and longest DNA fragments. To maximize the sensitivity of cf-DNA quantification, the ALU repeats were used as a target of the qPCR.

Two primer pairs were designed as follows: the first primer set (ALU244) was amplified only the longer (244 bp) DNA fragment, whereas the second primer set (ALU83) were amplified both the shorter (83 bp) and longer, because the ALU83 annealing site was within the ALU244 annealing site. The results obtained using the ALU83 primers represent the total cell free plasma circulating DNA, while the results obtained using the ALU244 primers reflect the amount of DNA released from non-apoptotic cells.

The probe sequence was 6FAM-CCTGGCCAACATGGTGAAACCCC-TMR.

The two reactions were performed in 20 $\mu$l final volume containing 1 $\mu$l of sample, 1X FluoCycle™ II Probe (Euroclone®, Milan, Italy), 0.25 $\mu$M of probe and 0.9 $\mu$M of each ALU244 primer in ones or 0.05 $\mu$M of forward and 0.9 $\mu$M of reverse primer for ALU83 in the other. The qPCR amplification consisted of an initial denaturation step for 5 minutes at 95°C, followed by 40 cycles of denaturation for 15 seconds at 95°C and annealing/extension for 1 minute at 62°C, using the 7300 Real Time PCR System and 7500 Fast Real Time PCR System (Applied Biosystems, Milan, Italy).

The samples were tested in triplicate and an appropriate negative reference (no template) samples were included. The absolute amount of cf-DNA in each sample was determined by a standard curve, using 10-fold serial dilutions (from 10 ng to 1 pg) of genomic DNA obtained from healthy subject buffy-coat.

**Claims**

1. An *in vitro* diagnostic method for prostate cancer comprising the step of determining the amount of the ALU 244 sequence and the amount of the ALU 83 sequence in an isolated sample of body fluid, determining a value which is the ratio between the amount of ALU 244 and of ALU 83 in the isolated sample of body fluid, wherein said ratio is compared to a reference value from healthy patients.

2. The *in vitro* diagnostic method according to claim 1, wherein the isolated sample of body fluid is purified in a purification step before determining the amount of the ALU sequence.

3. The *in vitro* diagnostic method according to any one of claim 1 or 2, wherein the isolated sample of the body fluid is treated in order to extract the DNA from the isolated sample before determining the amount of the ALU sequence.

4. The *in vitro* diagnostic method according to any one of the preceding claims, wherein said isolated sample of body fluid is a sample of blood, plasma, serum, saliva, urine, fluid from lymph nodes or lymph organs, bone marrow, fluid from pleura, breast ducts, lacrimal fluid, serous fluid, peritoneal fluid, cerebral spinal fluid, stool, sputum, ascites, gastric or pancreatic juice, sweat.

5. The *in vitro* diagnostic method according to any one of the preceding claims, wherein said body fluid is from a human or an animal.

6. The *in vitro* diagnostic method according to claim 5, wherein said body fluid is from dog.

7. The *in vitro* diagnostic method according to any one of the preceding claims, wherein said isolated sample has a volume comprised between about 300 $\mu$l-1 ml and preferably is about 500 $\mu$l.

8. The *in vitro* diagnostic method according to any one of the preceding claims, wherein the step of determining the amount of ALU 244 comprises the use of the following primers:

| | Sequence 5'→3' : | |
|---|---|---|
| forward | GCGGTGGCTCACGCCTGTAA | SEQ.ID. n.1 |
| reverse | GGAGTGCAGTGGCGCGATCT | SEQ. ID. n.2 |

9. The *in vitro* diagnostic method according to any one of claims 1 to 8, wherein the step of determining the amount of ALU 83 comprises the use of the following primers:

| | | |
|---|---|---|
| forward | CTGAGGTCAGGAGTTCGAGACC | SEQ.ID. n.3 |
| reverse | CCACGCCCGGCTAATTTT | SEQ. ID. n.4 |

10. The *in vitro* diagnostic method according to any one of the preceding claims, wherein the amount of ALU 244 and/or of ALU 83 is determined by quantitative real-time polymerase chain reaction.

11. Use of an isolated nucleotidic sequence having a sequence selected from the group comprising SEQ.ID. n. 1, 2, 3, 4 and 5 for the methods of any one of claims 1 to 10.

**Patentansprüche**

1. *In vitro* Diagnoseverfahren für Prostatakrebs umfassend den Schritt des Bestimmens der Menge der ALU 244 Sequenz und der Menge der ALU 83 Sequenz in einer isolierten Probe von Körperflüssigkeit, Bestimmen eines Wertes, der das Verhältnis zwischen der Menge an ALU 244 und ALU 83 in der isolierten Probe von Körperflüssigkeit darstellt, wobei das Verhältnis mit einem Referenzwert von gesunden Patienten verglichen wird.

2. *In vitro* Diagnoseverfahren gemäß Anspruch 1, wobei die isolierte Probe von Körperflüssigkeit vor Bestimmen der Menge der ALU Sequenz in einem Aufreinigungsschritt aufgereinigt wird.

3. *In vitro* Diagnoseverfahren gemäß einem der Ansprüche 1 oder 2, wobei die isolierte Probe von Körperflüssigkeit vor Bestimmen der Menge der ALU Sequenz behandelt wird, um die DNA aus der isolierten Probe zu extrahieren.

4. *In vitro* Diagnoseverfahren gemäß einem der vorhergehenden Ansprüche, wobei die isolierte Probe von Körperflüssigkeit eine Probe von Blut, Plasma, Serum, Speichel, Urin, Flüssigkeit aus Lymphknoten oder Lymphorganen, Knochenmark, Flüssigkeit aus der Pleura, Brustgängen, Tränenflüssigkeit, seröser Flüssigkeit, Peritonealflüssigkeit, zerebrospinaler Flüssigkeit, Stuhl, Auswurf, Aszites, Magen- oder Pankreassaft, Schweiß ist.

5. *In vitro* Diagnoseverfahren gemäß einem der vorhergehenden Ansprüche, wobei die Körperflüssigkeit von einem Menschen oder einem Tier ist.

6. *In vitro* Diagnoseverfahren gemäß Anspruch 5, wobei die Körperflüssigkeit vom Hund ist.

7. *In vitro* Diagnoseverfahren gemäß einem der vorhergehenden Ansprüche, wobei die isolierte Probe ein Volumen aufweist, das zwischen etwa 300 μl-1 ml liegt und bevorzugt etwa 500 μl ist.

8. *In vitro* Diagnoseverfahren gemäß einem der vorhergehenden Ansprüche, wobei der Schritt des Bestimmens der Menge an ALU 244 die Verwendung der folgenden Primer umfasst:

|  | Sequenz 5'→3': |  |
|---|---|---|
| vorwärts | GCGGTGGCTCACGCCTGTAA | SEQ. ID. n.1 |
| rückwärts | GGAGTGCAGTGGCGCGATCT | SEQ.ID. n.2 |

9. *In vitro* Diagnoseverfahren gemäß einem der Ansprüche 1 bis 8, wobei der Schritt des Bestimmens der Menge an ALU 83 die Verwendung der folgenden Primer umfasst:

| vorwärts | CTGAGGTCAGGAGTTCGAGACC | SEQ.ID. n.3 |
|---|---|---|
| rückwärts | CCACGCCCGGCTAATTTT | SEQ.ID. n.4 |

10. *In vitro* Diagnoseverfahren gemäß einem der vorhergehenden Ansprüche, wobei die Menge an ALU 244 und/oder an ALU 83 mittels quantitativer Echtzeit-Polymerase-Kettenreaktion bestimmt wird.

11. Verwendung einer isolierten nukleotidischen Sequenz, die eine Sequenz ausgewählt aus der Gruppe umfassend SEQ.ID. n. 1, 2, 3, 4 und 5 aufweist, für die Verfahren nach einem der Ansprüche 1 bis 10.

**Revendications**

1. Procédé *in vitro* de diagnostic du cancer de la prostate comprenant l'étape de détermination de la quantité de la séquence ALU 244 et de la quantité de la séquence ALU 83 dans un échantillon isolé de liquide corporel, de détermination d'une valeur qui est le rapport entre la quantité de ALU 244 et de ALU 83 dans l'échantillon isolé de liquide corporel, dans lequel ledit rapport est comparé à une valeur de référence provenant de patients en bonne santé.

2. Procédé *in vitro* de diagnostic selon la revendication 1, dans lequel l'échantillon isolé de liquide corporel est purifié dans une étape de purification avant la détermination de la quantité de la séquence ALU.

3. Procédé *in vitro* de diagnostic selon l'une quelconque de la revendication 1 ou 2, dans lequel l'échantillon isolé du liquide corporel est traité afin d'extraire l'ADN à partir de l'échantillon isolé avant la détermination de la quantité de la séquence ALU.

4. Procédé *in vitro* de diagnostic selon l'une quelconque des revendications précédentes, dans lequel ledit échantillon isolé de liquide corporel est un échantillon de sang, de plasma, de sérum, de salive, d'urine, de liquide des ganglions lymphatiques ou des organes lymphatiques, de moelle osseuse, de liquide de la plèvre, des canaux mammaires, de liquide lacrymal, de liquide séreux, de liquide péritonéal, de liquide céphalo-rachidien, de selles, de crachats, d'ascite, de suc gastrique ou pancréatique, de sueur.

5. Procédé *in vitro* de diagnostic selon l'une quelconque des revendications précédentes, dans lequel ledit liquide corporel provient d'un être humain ou d'un animal.

6. Procédé *in vitro* de diagnostic selon la revendication 5, dans lequel ledit liquide corporel provient d'un chien.

7. Procédé *in vitro* de diagnostic selon l'une quelconque des revendications précédentes, dans lequel ledit échantillon isolé a un volume compris entre environ 300 μl et 1 ml et est de préférence d'environ 500 μl.

8. Procédé *in vitro* de diagnostic selon l'une quelconque des revendications précédentes, dans lequel l'étape de détermination de la quantité de ALU 244 comprend l'utilisation des amorces suivantes :

> Séquence 5' → 3' :
> directe    GCGGTGGCTCACGCCTGTAA    SEQ ID NO : 1
> inverse    GGAGTGCAGTGGCGCGATCT    SEQ ID NO : 2

9. Procédé *in vitro* de diagnostic selon l'une quelconque des revendications 1 à 8, dans lequel l'étape de détermination de la quantité de ALU 83 comprend l'utilisation des amorces suivantes :

> directe    CTGAGGTCAGGAGTTCGAGACC    SEQ ID NO : 3
> inverse    CCACGCCCGGCTAATTTT    SEQ ID NO : 4

10. Procédé *in vitro* de diagnostic selon l'une quelconque des revendications précédentes, dans lequel la quantité de ALU 244 et/ou de ALU 83 est déterminée par une réaction en chaîne par polymérase en temps réel quantitative.

11. Utilisation d'une séquence nucléotidique isolée ayant une séquence du groupe comprenant SEQ ID NO : 1, 2, 3, 4 et 5 pour les procédés selon l'une quelconque des revendications 1 à 10.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MARIANGELA ZANE et al.** Circulating cell-free DNA, SLC5A8 and SLC26A4 hypermethylation, BRAFV600E: A non-invasive tool panel for early detection of thyroid cancer. *Biomedicine & Pharmacotherapy,* 01 October 2013, vol. 67 (8), 723-730

- Plasma cell-free DNA and its DNA integrity as biomarker to distinguish prostate cancer from benign prostatic hyperplasia in patients with increased serum prostate-specific antigen. **FENG JIANG et al.** International urology and nephrology. Akademiai, Budapest, 19 June 2013, vol. 45, 1023-1028